(19) Europäisches Patentamt
European Patent Office
Office européen des brevets

(11) Veröffentlichungsnummer: **0 236 700 B1**

# (12) EUROPÄISCHE PATENTSCHRIFT

(45) Veröffentlichungstag der Patentschrift: **09.12.92**

(51) Int. Cl.5: **C07C 47/232**, C07C 45/00, C07D 303/14, C07D 301/12

(21) Anmeldenummer: **87100772.0**

(22) Anmeldetag: **21.01.87**

## (54) Alpha,beta-substituierte Acroleine, Verfahren zu deren Herstellung und deren Verwendung.

(30) Priorität: **23.01.86 DE 3601927**

(43) Veröffentlichungstag der Anmeldung: **16.09.87 Patentblatt 87/38**

(45) Bekanntmachung des Hinweises auf die Patenterteilung: **09.12.92 Patentblatt 92/50**

(84) Benannte Vertragsstaaten: **CH DE FR GB IT LI**

(56) Entgegenhaltungen:
**EP-A- 0 024 611**
**EP-A- 0 094 564**
**CH-A- 133 680**
**DE-B- 1 921 560**
**DE-B- 2 803 791**
**US-A- 2 102 965**
**US-A- 3 920 755**
**US-A- 4 435 585**

(73) Patentinhaber: **BASF Aktiengesellschaft**
**Carl-Bosch-Strasse 38**
**W-6700 Ludwigshafen(DE)**

(72) Erfinder: **Janssen, Bernd, Dr.**
**Leuschnerstrasse 18 a**
**W-6700 Ludwigshafen(DE)**
Erfinder: **Karbach, Stefan, Dr.**
**Sperlinggasse 3**
**W-6700 Ludwigshafen(DE)**
Erfinder: **Recker, Hans-Gert, Dr.**
**Lisztstrasse 117**
**W-6700 Ludwigshafen(DE)**
Erfinder: **Thyes, Marco, Dr.**
**Thorwaldsenstrasse 1**
**W-6700 Ludwigshafen(DE)**

EP 0 236 700 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft alpha,beta-substituierte Acroleine, ein Verfahren zu deren Herstellung und deren Verwendung zur Herstellung von Hydroxymethyloxiranen.

Hydroxymethyloxirane sind wertvolle Zwischenprodukte zur Synthese antimykotisch und fungizid wirksamer Azolylmethyloxirane. Derartige Azolylmethyloxirane sind in der europäischen Patentanmeldung 94 564 der Anmelderin beschrieben. Diese Verbindungen sind erhältlich, indem man aus Hydroxymethyloxiranen zugängliche Halogenmethyloxirane mit den entsprechenden Triazolen oder Imidazolen oder den reaktiven Derivaten dieser Verbindungen umsetzt. Nach der Lehre der EP-A-94 564 lassen sich die Hydroxymethyloxirane durch Epoxidierung der entsprechenden Allylalkohole darstellen.

Die Herstellung von Hydroxymethyloxiranen durch Epoxidierung der entsprechenden Allylalkohole ist auch in anderen Druckschriften beschrieben, so beispielsweise in Houben-Weyl, VI/3, 371, im Journal of Organic Chemistry 30, 2074 (1965), in J. Am. Chem. Soc. 102, 5974 (1980) und J. Am. Chem. Soc. 95, 6136 (1973).

Die bekannten Verfahren sind insofern nachteilig, als bei der Epoxidierung infolge thermischer Belastung häufig E/Z-Isomerengemische beobachtet werden. Außerdem sind die entsprechenden Allylalkohole teilweise nur sehr schwer zugänglich.

US-A 4 435 585 betrifft u.a. in 4-Stellung des Phenylrings mit Alkyl- und Methoxygruppen substituierte 2-Methylpropiophenone. Die Verbindungen werden aus den entsprechenden Arylketonen in mehreren Schritten, deren einer eine Hydroformylierung ist, hergestellt. In DE-B 1 921 560 wird 4-Methyl-2-phenyl-2-pentenal durch die Aldolkondensation von Phenylacetaldehyd mit Isobutyraldehyd synthetisiert. In US-A 2 102 965 wird p-Isopropyl-$\alpha$-methylzimtaldehyd durch die Aldolkondensation von 4-Isopropyl-benzaldehyd (Cuminaldehyd) mit dem Bisulfitaddukt von Propionaldehyd erzeugt. In US-A 3 313 843 werden diverse $\alpha$-Alkyl-alkoxy-zimtaldehyde als Ausgangsmaterialien für die Herstellung von Zimtsäureestern genannt, ohne daß eine Methode zu deren Herstellung angegeben wird.

Der Erfindung liegt die Aufgabe zugrunde, neue alpha,beta-substituierte Acroleine, sowie ein Verfahren zu deren Herstellung zur Verfügung zu stellen, aus denen sich Hydroxymethyloxirane stereo-selektiv und in sehr hohen Ausbeuten darstellen lassen.

Diese Aufgabe wird gelöst durch Bereitstellung von alpha,beta-substituierten Acroleinen der allgemeinen Formel I

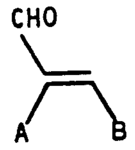

I,

worin A und B, die gleich oder verschieden sind, für $C_1$-$C_4$-Alkyl, Naphthyl, Biphenyl oder Phenyl, das durch ein oder mehrere Halogen-, Nitro-, $C_1$-$C_4$-Alkyl-, $C_1$-$C_4$-Alkoxi, $C_1$-$C_4$-Halogenalkyl-, Phenoxi- oder Phenylsulfonylreste substituiert sein kann, stehen.

Das Verfahren zur Herstellung der alpha-beta-substituierten Acroleine der allgemeinen Formel I ist dadurch gekennzeichnet, daß man eine Verbindung der allgemeinen Formel III

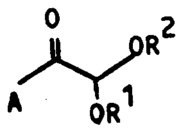

(III),

worin A die vorstehenden Bedeutungen besitzt und $R^1$ und $R^2$ gleich oder verschieden sind und für $C_1$-$C_4$-Alkyl stehen oder zusammengenommen die zur Vervollständigung eines Rings erforderlichen Kohlenstoffatome aufweisen, mit einer Phosphorverbindung der allgemeinen Formeln IV oder V

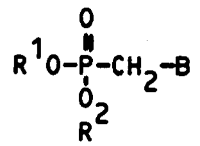 (IV) 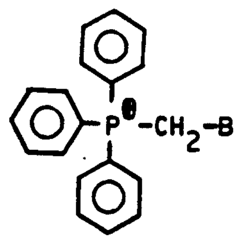 $X^{\ominus}$ (V),

worin B die vorstehend angegebenen Bedeutungen besitzt, $R^1$ und $R^2$ wie oben definiert sind und $X^{\ominus}$ für ein Halogenidion steht, in Gegenwart einer Base umsetzt.

Die Weiterverarbeitung der erfindungsgemäßen alpha-beta-substituierten Acroleine erfolgt dadurch, daß man sie zu den entsprechenden Formyloxiranen epoxidiert und diese unmittelbar, d.h. in einer Eintopfreaktion, in der Reaktionsmischung zu den Hydroxymethyloxiranen der nachfolgenden Formel II

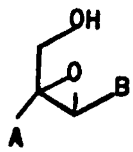 (II),

worin A und B die vorstehend genannten Bedeutungen besitzen, reduziert.

Es wurde überraschend festgestellt, daß man aus den erfindungsgemäßen alpha-beta-substituierten Acroleinen die Hydroxymethyloxirane in sehr hohen Ausbeuten erhält, wobei diese Verbindungen teilweise stereoselektiv gebildet werden.

Die Umsetzung der erfindungsgemäßen alpha-beta-substituierten Acroleine zu den Methyloxiranen erfolgt gewünschtenfalls in Gegenwart eines Lösungs- oder Verdünnungsmittels und gewünschtenfalls unter Zusatz einer organischen oder anorganischen Base als Katalysator.

Zu den bevorzugten Lösungsmitteln gehören Alkohole wie z.B. Methanol, Ethanol, Isopropanol, n-Propanol, n-Butanol, iso-Butanol, tertiär-Butanol, Cyclohexanol sowie halogenierte Kohlenwasserstoffe wie z.B. Methylenchlorid, Chloroform, Tetrachlorkohlenstoff, Dichlorethan, Chlorbenzol, aber auch Kohlenwasserstoffe wie Pentan, Hexan, Heptan, Cyclohexan, Toluol und Xylol sowie Amide wie z.B. Dimethylacetamid und N-Methylpyrrolidon. Weitere geeignete Lösungsmittel sind Nitrile wie z.B. Acetonitril oder Sulfoxide wie z.B. Dimethylsulfoxid. Auch Harnstoffderivate, z.B. 1,3-Dimethyl-3,4,5-tetrahydro-2 (1H)-pyrimidinon (DMPU) sind verwendbar. Man kann aber auch vorteilhaft Gemische dieser Lösungsmittel einsetzen. Gegebenenfalls wird unter Zusatz eines Phasentransferkatalysators, z.B. der von E.V. Dehmlow, S.S. Dehmlow - Phase Transfer Catalysis (1980), Verlag Chemie - beschriebenen Phasentransferkatalysatoren gearbeitet.

Geeignete Basen sind beispielsweise Alkalihydroxide, z.B. Natrium- oder Kaliumhydroxid, Alkalicarbonate, z.B. Natrium- oder Kaliumcarbonat, ferner Alkoholate wie z.B. Natrium- oder Kaliummethylat, -ethylat, -propylat, -isopropylat, -n-butanolat, -isobutanolat, -tertiär-butanolat, -cyclohexanolat sowie tertiäre Amine, z.B. Trialkylamine, wobei die Alkylreste gleich oder verschieden sind und Methyl, Ethyl, Propyl, iso-Propyl, n-Butyl, iso-Butyl, tertiär-Butyl oder Cyclohexyl sein können. Auch aromatische Amine, z.B. Pyridin oder N,N′-Dimethylaminopyridin sind einsetzbar. Besonders bevorzugt sind Natrium- und Kaliumhydroxid und -carbonat.

Eine für die Reaktion zweckmäßige Menge an basischem Katalysator beträgt beispielsweise 0,1 bis 20 Mol-%, vorzugsweise 0,5 bis 10 Mol-%, bezogen auf das eingesetzte alpha-beta-substituierte Acrolein der allgemeinen Formel II.

Für die Epoxidierung eignet sich 3 % bis 80 %ige wäßrige Wasserstoffperoxidlösung, insbesondere 20 % bis 60 %ige Wasserstoffperoxidlösungen, besonders bevorzugt sind 30%ige wäßrige Lösungen von Wasserstoffperoxid.

Als Reduktionsmittel kommen Metallhydride wie z.B. Diisobutylaluminiumhydrid, Natrium-, Lithium- und Kaliumhydrid, -borhydrid oder -cyanoborhydrid, aber auch Lithiumaluminiumhydride der allgemeinen Formel

$$Li\ Al\ (H)_m\ (OR)_n$$

mit m = 1 bis 4 und n = 4 - m, wobei R gleich oder verschieden sein kann und allgemein für Alkylreste, z.B. Methyl, Ethylk, Propyl, iso-Propyl, n-Butyl, iso-Butyl, tert.-Butyl oder Cyclohexyl steht, oder Wasserstoff, gegebenenfalls in Gegenwart eines geeigneten Katalysators, z.B. Rhodium oder Ruthenium, in Frage. Besonders bevorzugt ist Natriumborhydrid.

Die Reaktion wird im allgemeinen bei Temperaturen zwischen -20 °C und +120 °C, bevorzugt bei -5 °C bis +50 °C, besonders bevorzugt bei 0 °C - +30 °C drucklos oder unter Druck, kontinuierlich oder diskontinuierlich durchgeführt.

Für die Epoxidierung werden in der Regel äquimolare Mengen des alpha,beta-substituierten Acroleins und $H_2O_2$ zur Reaktion gebracht. Man kann jedoch auch einen Überschuß an $H_2O_2$, z.B. 1 % - 20 % verwenden.

Für die Reduktion werden bevorzugt stöchiometrische Mengen des Reduktionsmittels eingesetzt, man kann jedoch auch einen Überschuß, beispielsweise 0,5 % - 20 % verwenden.

Nach Beendigung der Umsetzung kann das Endprodukt auf übliche Weise, z.B. durch Extraktion mit geeigneten organischen Lösungsmitteln, z.B. chlorierten Kohlenwasserstoffen, Kohlenwasserstoffen, Estern, Ethern oder besonders bevorzugt durch direkte kristallisation aus der Reaktionsmischung isoliert werden, gegebenenfalls durch Zugabe von Wasser.

Die schnelle und fast quantitative Umsetzung der alpha-beta-substituierten Acroleine der allgemeinen Formel II zu den Hydroxymethyloxiranen der allgemeinen Formel I ist insofern überraschend, als die intermediär gebildeten Epoxyaldehyde sehr instabil sind und leicht zu den entsprechenden Säuren bzw. Spaltprodukten durch Öffnung des Oxiranrings weiter reagieren.

Durch die bei der erfindungsgemäßen Verwendung beschriebene einstufige Verfahrensweise kann hier jedoch die Isolierung dieser labilen Zwischenstufe umgangen werden. Eine technisch vorteilhafte Durchführung dieses Verfahrens ergibt sich durch wesentliche Vereinfachungen im Aufarbeitungsschritt in dem ein Extraktions- und Reinigungsprozeß entfallen kann, da im allgemeinen das Endprodukt direkt aus der Reaktionsmischung auskristallisiert.

In den Verbindungen der allgemeinen Formeln I und II stehen die Reste A und B vorzugsweise für Methyl, Ethyl, Propyl, Isopropyl, Butyl, sek.-Butyl, iso-Butyl, tert.-Butyl, 1-Naphthyl, 2-Naphthyl, p-Biphenyl, Phenyl, 2-Chlorphenyl, 3-Chlorphenyl, 4-Chlorphenyl, 2-Fluorphenyl, 3-Fluorphenyl, 2-Fluor-6-Chlorphenyl, 4-fluorphenyl, 4-Bromphenyl, 2,4-Dichlorphenyl, 3,4-Dichlorphenyl, 3,5-Dichlorphenyl, 2,6-Dichlorphenyl, 3-Chlor-4-methylphenyl, 2-Methoxyphenyl, 3-Methoxyphenyl, 2,4-Dimethoxyphenyl, 3,4-Dimethoxyphenyl, 4-Methoxyphenyl, 4-Ethoxyphenyl, 4-tert-Butoxyphenyl, 4-Methylphenyl, 4-Ethylphenyl, 4-Isopropylphenyl, 4-tert.-Butylphenyl, 4-Phenoxyphenyl, 3-Phenoxyphenyl, 3-Nitrophenyl, 4-Nitrophenyl, 3-Trifluormethylphenyl, 4-Trifluormethylphenyl oder 4-Phenylsulfonylphenyl.

Besonders bevorzugt bedeuten die Reste A und B in den Verbindungen der allgemeinen Formeln I und II Methyl, tert.-Butyl, Phenyl, 2-Chlorphenyl, 4-Chlorphenyl, 2-Fluorphenyl, 3-Fluorphenyl, 4-Fluorphenyl, 2-Fluor-6-Chlorphenyl, 4-Bromphenyl, 2,4-Dichlorphenyl oder 4-tert.-Butylphenyl.

Im Stand der Technik sind Verfahren zur Herstellung phenylsubstituierter Acroleine der allgemeinen Formel I entsprechenden Typs bekannt, die jedoch nur schlechte Ausbeuten liefern.

So führt beispielsweise die Kondensation des sehr schwer handhabbaren Phenylacetaldehyds mit Benzaldehyd (15 % Überschuß) in nur etwa 70 % Ausbeute zum alpha-Phenylzimtaldehyd (Alder et al, Ann. d. Chemie 596, 128 (1954). Aufgrund der häufigen Bildung unübersichtlicher, oft schwer trennbarer Produktgemische sind diese gemischten Aldolkondensationen technisch von sehr geringem Interesse. (A. T. Nielsen, W. J. Houlihar Org. Reactions 16, (1968); Houben-Weyl VII/1, 76). Speziell die Synthese der acceptorsubstituierten alpha-Phenylacroleine ist auf diesem Wege äußerst unbefriedigend, da sowohl die Darstellung als auch Handhabung der entsprechenden acceptorsubstituierten Phenylacetaldehyde mit extremen Schwierigkeiten verbunden ist.

Auch die Umsetzung von Zimtaldehyd mit aromatischen Diazoniumsalzen, z.B. p-Chlorbenzoldiazoniumchlorid, zu alpha-phenylsubstituierten Zimtaldehyden liefert mit etwa 35 % nur äußerst unbefriedigende Ausbeuten. (H. Meerwein et al, J. prakt. Chemie 152, 1935, Org. Reactions 24, 225 (1973).

Erfindungsgemäß wird ein Verfahren zur Herstellung der alpha-beta-substituierten Acroleine der allgemeinen Formel I zur Verfügung gestellt, das dadurch gekennzeichnet ist, daß man eine Verbindung der allgemeinen Formel III

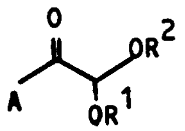

(III),

worin A die in den Ansprüchen 1 bis 3 angegebenen Bedeutungen besitzt und $R^1$ und $R^2$ gleich oder verschieden sind und für $C_1$-$C_4$-Alkyl stehen oder zusammengenommen die zur Vervollständigung eines Rings erforderlichen Kohlenstoffatome aufweisen, mit einer Phosphorverbindung der allgemeinen Formeln IV oder V

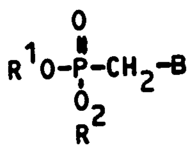

(IV)

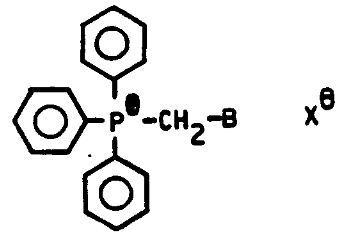

(V),

worin B die in den Ansprüchen 1 bis 3 angegebenen Bedeutungen besitzt, $R^1$ und $R^2$ wie oben definiert sind und $X^{\ominus}$ für ein Halogenidion steht, in Gegenwart einer Base umsetzt.

Diese Umsetzung erfolgt mit sehr hohen Ausbeuten und teilweise stereoselektiv. Die Ausgangsverbindungen der allgemeinen Formel III sind beispielsweise in der DE-A 34 07 005 und teilweise in der deutschen Patentanmeldung P 35 39 629.6 der Anmelderin beschrieben.

In den Verbindungen der allgemeinen Formeln III, IV und V besitzen die Reste A und B dieselben Bedeutungen wie in den Verbindungen der allgemeinen Formeln I und II. Vorzugsweise stehen die Reste A und B für Methyl, Ethyl, Propyl, Isopropyl, Butyl, sek.-Butyl, iso-Butyl, tert.-Butyl, 1-Naphthyl, 2-Naphthyl, p-Biphenyl, Phenyl, 2-Chlorphenyl, 3-Chlorphenyl, 4-Chlorphenyl, 2-Fluorphenyl, 3-Fluorphenyl, 2-Fluor-6-Chlorphenyl, 4-fluorphenyl, 4-Bromphenyl, 2,4-Dichlorphenyl, 3,4-Dichlorphenyl, 3,5-Dichlorphenyl, 2,6-Dichlorphenyl, 3-Chlor-4-methylphenyl, 2-Methoxyphenyl, 3-Methoxyphenyl, 2,4-Dimethoxyphenyl, 3,4-Dimethoxyphenyl, 4-Methoxyphenyl, 4-Ethoxyphenyl, 4-tert.-Butoxyphenyl, 4-Methylphenyl, 4-Ethylphenyl, 4-Isopropylphenyl, 4-tert.-Butylphenyl, 4-Phenoxyphenyl, 3-Phenoxyphenyl, 3-Nitrophenyl, 4-Nitrophenyl, 3-Trifluormethylphenyl, 4-Trifluormethylphenyl oder 4-Phenylsulfonylphenyl.

Besonders bevorzugt stehen die Reste A und B für Methyl, tert.-Butyl, Phenyl, 2-Chlorphenyl, 4-Chlorphenyl, 2-Fluorphenyl, 3-Fluorphenyl, 4-Fluorphenyl, 2-Fluor-6-Chlorphenyl, 4-Bromphenyl, 2,4-Dichlorphenyl oder 4-tert.-Butylphenyl.

Das Verfahren zur Herstellung der erfindungsgemäßen alpha-beta-substituierten Acroleine wird gegebenenfalls in Gegenwart eines Lösungs- oder Verdünnungsmittels und besonders zweckmäßig bei Temperaturen zwischen -10 °C und +130 °C in Gegenwart einer Base durchgeführt.

Zu den bevorzugten Lösungsmitteln gehören Alkohole, z.B. Methanol, Ethanol, Propanol, iso-Propanol, n-Butanol, iso-Butanol, tert.-Butanol und Cyclohexanol sowie Kohlenwasserstoffe wie z.B. Pentan, Hexan, Heptan, Toluol, Xylol oder Cyclohexan, weiterhin halogenierte Kohlenwasserstoffe z.B. Methylenchlorid, Chloroform, Tetrachlorkohlenstoff, Dichlorethan oder Chlorbenzol, aber auch Ether, wie z.B. Diethylether, Methyl-tert.-Butylether, Glykoldimethyl- und diethylether sowie Dioxan und THF. Besonders bevorzugt sind polar aprotische Lösungsmittel, z.B. Dimethylsulfoxid, Hexamethylphosphorsäuretriamid, Acetonitril, N-Methylpyrrolidon, Dimethylacetamid oder 1,3-Dimethyl-3,4,5-tetrahydro-2(1H)-pyrimidine n (DMPU). Ganz besonders bevorzugt ist Dimethylformamid.

Die Umsetzung erfolgt zweckmäßig in einem Temperaturbereich von -10 °C bis +130 °C, bevorzugt bei 0 °C - 60 °C, besonders bevorzugt bei Temperaturen zwischen 10 ° und +30 °C.

Als Basen eignen sich beispielsweise Alkalihydride wie Lithium-, Natrium-und Kaliumhydrid, Alkaliamide wie Natrium- und Kaliumamid oder Lithium-diisopropylamid,ferner Alkalialkoholate, z.B. Natrium- und Kalium-tert.-butoxid, Natrium- und Kalium-methylat und -ethylat sowie Natrium-oder Kaliumtriphenylmethyl und Naphthalin-lithium, -natrium und -kalium.

Bevorzugt verwendet werden die Alkalialkoholate, besonders bevorzugt Natrium- und Kalium-methylat, -ethylat, -tert.butylat und -isopropylat.

Bei der Durchführung des Verfahrens setzt man vorzugsweise äquimolare Mengen des Ketons der allgemeinen Formel III und der Phosphorverbindung der allgemeinen Formel IV bzw. V ein.

Man kann auch einen Überschuß an einem Reaktionsteilnehmer, beispielsweise an dem billigen Reaktionsteilnehmer, z.B. einen Überschuß bis zu 30 % verwenden.

Zur Isolierung der alpha-beta-substituierten Acroleine der allgemeinen Formel II wird üblicherweise mit wäßriger Mineralsäure, z.B. HCl oder $H_2SO_4$ versetzt und das Endprodukt gegebenenfalls mit einem geeigneten Lösungsmittel, z.B. Kohlenwasserstoffe, chlorierte Kohlenwasserstoffe, Ester, Ketone oder Ether aus der Reaktionsmischung extrahiert. Besonders bevorzugt wird die Verbindung der Formel II direkt aus der Reaktionsmischung gegebenenfalls unter Zugabe von Wasser auskristallisiert.

Die Ausgangsverbindung der allgemeinen Formel IV erhält man üblicherweise durch Umsetzung des entsprechenden Halogenids mit einem Phosphorigsäurealkylester (Houben-Weyl XII/1, 433; G.M. Kosolapoff, Org. Reactions 6, 276 (1951). Die so erhaltenen Phosphonate können sowohl gereinigt als auch als Rohprodukt in der Wittig-Horner-Reaktion eingesetzt werden.

Die Phosphoniumsalze der allgemeinen Formel V sind über die Reaktion von Triphenylphosphin mit den entsprechenden Halogeniden zugänglich (A. Maerckier, Org. Reactions 14, 270 (1965); G. Wittig, Angew. Chemie 68, 505 (1956); Houben-Weyl V/2 a, 185).

Die omega,omega′-Bisalkoxyketone der allgemeinen Formel III sind z.B. durch übliche Umsetzung von Methylketonen mit Sulfurylchlorid oder anderen gängigen Chlorierungsmitteln zu omega-Dichlorketonen, anschließendem Chlorid-Austausch mit Alkalialkoholat in dem entsprechenden Alkohol als Lösungsmittel sowie direkter Umacetalisierung mit konzentrierter Mineralsäure, z.B. HCl erhältlich (DE 34 07 005 Al).

Die Erfindung wird durch die nachfolgenden Beispiele weiter erläutert.

I. Herstellung der Hydroxymethyloxirane

Beispiel 1

1-Hydroxy-methyl-1-(4-Chlorphenyl)-2-phenyl-oxiran

Zu einer Lösung von 121 g (E)-alpha-(4-Chlorphenyl) Zimtaldehyd in 400 ml Methanol und 3 ml 25%iger NaOH werden bei 5°C - 10°C 70 g 3O%iges $H_2O_2$ zugetropft. Nach Beendigung der Zugabe wird 1 h bei RT nachgerührt. Anschließend werden bei 10° - 20°C 4.625 g $NaBH_4$ zugegeben. Es wird 1 h bei RT nachgerüht. Nach Zugabe von 500 ml $H_2O$ kristallisieren 116 g (89.2 %) 1-Hydroxymethyl-1-(4-Chlorphenyl)-2-phenyloxiran vom Schmelzpunkt 90 - 96°C.

Beispiel 2

(Z)-2-(4-Chlorphenyl)-3-(2,4-dichlorphenyl)-2-(hydroxymethyl)oxiran

Zu dem Gemisch aus 0,65 g (0,005 mol) Kaliumcarbonat und 65 ml Methanol werden 13,5 g (0,043 mol) (E)-2-(4-Chlorphenyl-3-(2,4-dichlorphenyl)acrolein gegeben. Anschließend werden unter Rühren und unter Stickstoff innerhalb ca. 15 Min. bei 0 bis 5°C 4,5 ml (5,0 g; 0,044 mol) einer 30%igen Lösung von Wasserstoffperoxid in Wasser zugetropft. Die Suspension wird 30 Min. bei 0 bis 5°C unter $N_2$ nachgerührt und die Temperatur unter Rühren auf ca. 25°C ansteigen gelassen. Unter Rühren wird innerhalb ca. 10 Min. bei 20 bis 30°C mit 0,95 g (0,025 mol) Natriumborhydrid versetzt. Anschließend wird noch 1 h bei ca. 25°C gerührt und dann 200 ml destilliertes Wasser hinzugefügt. Man erhält 13,3 g (0,040 mol; 93 % d.Th.) farblose Kristalle vom Schmelzpunkt 122 bis 127°C, bei denen es sich laut $^1$H-NMR-Spektrum um ein Gemisch aus (Z) und (E) im Verhältnis von ca. 85:15 handelt.

Dieses Gemisch aus (Z)- und (E)-2-(4-Chlorphenyl)-3-(2,4-dichlorphenyl)-2-(hydroxymethyl)-oxiran (Z:E = 85:15) wird aus 150 ml eines Methanol/destilliertes Wasser-Gemisches (Methanol/destilliertes Wasser = 9:1) umkristallisiert. Es werden 9,0 g (0,027 mol; 64 %) (Z)-2-(4-Chlorphenyl)-3-(2,4-dichlorphenyl)-2-(hydroxymethyl)oxiran von Schmelzpunkt 137 bis 140°C erhalten.

II. Herstellung der alpha-beta-substituierten Acroleine

Beispiel 3

(E)-(4-Chlorphenyl)Zimtaldehyd

Zu einer Lösung aus 270 g Kalium-tert.-butoxid in 2 l DMF wird bei 10° - 20°C unter Kühlung eine Mischung aus 500 g Benzylphosphorigsäurediethylester und 428 g omega,omega′-Bismethoxy-4-chloracetophenon zugetropft. Nach Beendigung der Zugabe wird 1 l 2n HCl unter Eiskühlung bei 10° - 25°C zudosiert. Nach 1/2 Stunde werden weitere 4 l Eiswasser zugegeben und der ausgefallene Festkörper abgesaugt. Auswaage 480 g (99 %) (E)-alpha(4-Chlorphenyl)zimtaldehyd vom Schmelzpunkt 70- 75°C.

Beispiel 4

(E)-2-(4-Chlorphenyl)-3-(2,4-dichlorphenyl)acrolein

Zu einem Gemisch aus 1000 ml Dimethylformamid und 216 g (1,20 mol) einer 30%igen Lösung von Natriummethylat in Methanol werden unter Rühren innerhalb 10 Min. bei 20 bis 24°C 214 g (1,00 mol) p-Chlor omega-omega-dimethoxyacetophenon getropft. Nach beendetem Zutropfen werden noch unter Rühren innerhalb 30 Min. bei 20 bis 30°C 312 g (1,05 mol) (2,4-Dichlorbenzyl)-phosphorsäure-diethylester zugegeben. Anschließend wird 3,5 h bei ca. 25°C nachgerührt und das Reaktionsgemisch dann mit 2 l-destilliertem Wasser versetzt. Es wird nochmals 30 Min. gerührt und danach abgesaugt. Nach dem Waschen mit destilliertem Wasser und dem Trocknen im Vakuumtrockenschrank bei 50°C werden 350 g (0,98 mol; 98 % d.Th.) (E)-2-(4-Chlorphenyl)-1-(2,4-dichlorphenyl)-3,3-dimethoxyprop-1-en als leicht gelbliche Kristalle vom Schmelzpunkt 87 bis 90°C erhalten.

Anschließend wird ein Gemisch aus 1000 ml destilliertem Wasser, 500 ml Methanol, 50 ml (59,7 g; 0,62 mol) konzentrierter Salzsäure und 350 g (0,98 mol) (E)-2-(4-Chlorphenyl)-1-(2,4-dichlorphenyl)-3,3-dimethoxyprop-1-en (I) unter Rühren 4 h bei Rückflußtemperatur gehalten. Es wird auf Raumtemperatur abgekühlt und dann unter Rühren 1000 ml Wasser zugegeben. Es werden 300 g (0,96 mol; 98 % d.Th.) (E)-2-(4-Chlorphenyl)-3-(2,4-dichlorphenyl)acrolein als leicht gelbliche Kristalle vom Schmelzpunkt 106 bis 109°C isoliert.

Beispiele 5 bis 67

Entsprechend den Beispielen 1 bis 4 wurden bzw. können die in der nachstehenden Tabelle aufgeführten Verbindungen hergestellt werden. Die als Öl oder Harz anfallenden Alkohole wurden teilweise über die entsprechenden Tosylate charakterisiert.

<u>Tabelle</u>

| Beispiel No. | A | B | 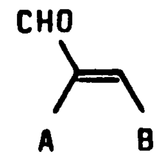 | 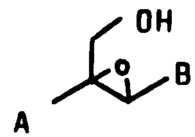 | 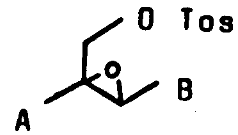 |
|---|---|---|---|---|---|
| 5 | $C_6H_5$ | $2\text{-F-}C_6H_4$ | Öl | Öl | 85 - 88° |
| 6 | " | $3\text{-F-}C_6H_4$ | | | |
| 7 | " | $4\text{-F-}C_6H_4$ | | | |
| 8 | " | $2\text{-F, }6\text{-Cl-}C_6H_3$ | | | |
| 9 | " | $2\text{-Cl-}C_6H_4$ | | | |
| 10 | " | $4\text{-Br-}C_6H_4$ | 170-173 | | |
| 11 | 4(Phenyl)-$C_6H_4$ | $2\text{-Cl-}C_6H_4$ | 106-108 | 153-155 | 99 |
| 12 | $2,4\text{-}Cl_2\text{-}C_6H_3$ | $C_6H_5$ | | | |
| 13 | " | $2\text{-F-}C_6H_4$ | 85 - 84 | Harz | |
| 14 | " | $3\text{-F-}C_6H_4$ | | | |
| 15 | " | $4\text{-F-}C_6H_4$ | | | |
| 16 | " | $2\text{-F, }6\text{-Cl-}C_6H_3$ | | | |
| 17 | " | $2\text{-Cl-}C_6H_4$ | | | |
| 18 | " | $3\text{-Cl-}C_6H_4$ | | | |
| 19 | " | $4\text{-Cl-}C_6H_4$ | | | |
| 20 | " | $2,4\text{-}Cl_2\text{-}C_6H_3$ | | | |
| 21 | " | $2,6\text{-}Cl_2\text{-}C_6H_3$ | | | |
| 22 | $2\text{-Cl-}C_6H_4$ | $C_6H_5$ | | | |
| 23 | " | $2\text{-F-}C_6H_4$ | Harz | | |
| 24 | " | $3\text{-F-}C_6H_4$ | Harz | | |
| 25 | " | $4\text{-F-}C_6H_4$ | Harz | | |

Tabelle

| Belspiel No. | A | B | 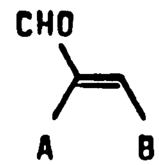 | 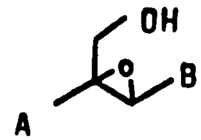 | 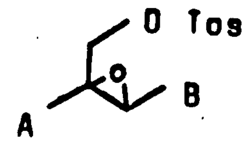 |
|---|---|---|---|---|---|
| 26 | " | 2-Cl-$C_6H_4$ | 87 | Harz | 128-130 |
| 27 | " | 3-Cl-$C_6H_4$ | Harz | | |
| 28 | " | 4-Cl-$C_6$-$H_4$ | | | |
| 29 | " | 2,4-$Cl_2$-$H_3$ | | | |
| 30 | " | 2,6-$Cl_2$-$C_6H_3$ | | | |
| 31 | " | 4-Br-$C_6H_4$ | | | |
| 32 | 2-F-$C_6H_4$ | $C_6H_5$ | | | |
| 33 | " | 2-F-$C_6H_4$ | | | |
| 34 | " | 3-F-$C_6H_4$ | | | |
| 35 | " | 4-F-$C_6H_4$ | | | |
| 36 | " | 2-Cl-$C_6H_4$ | 106-109 | | |
| 37 | " | 3-Cl-$C_6H_4$ | | | |
| 38 | " | 4-Cl-$C_6H_4$ | | | |
| 39 | " | 2,4-$Cl_2$-$C_6H_3$ | | | |
| 40 | 4-Br-$C_6H_4$ | 2-Cl-$C_6H_4$ | | | |
| 41 | 4-F-$C_6H_4$ | $C_6H_5$ | | | |
| 42 | " | 2-F-$C_6H_4$ | 89-92 | | |
| 43 | " | 3-F-$C_6H_4$ | | | |
| 44 | " | 4-F-$C_6H_4$ | | | |
| 45 | " | 2-F, 4-Cl-$C_6H_3$ | | | |
| 46 | " | 2-Cl-$C_6H_4$ | 85-88 | 103-105 | 104-105 |

**Tabelle**

| Beispiel No. | A | B | 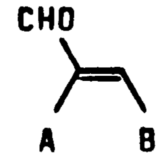 | 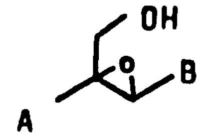 | 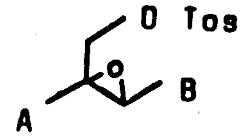 |
|---|---|---|---|---|---|
| 47 | " | 3-Cl-$C_6H_4$ | | | |
| 48 | " | 4-Cl-$C_6H_4$ | | | |
| 49 | " | 2,4-$Cl_2$-$C_6H_3$ | 98-101 | 130 | 116-117 |
| 50 | " | 2,6-$Cl_2$-$C_6H_3$ | | | |
| 51 | " | 4-Br-$C_6H_4$ | | | |
| 52 | 4-$CH_3$-$C_6H_4$ | $C_6H_5$ | | | |
| 53 | | 2-F-$C_6H_4$ | 75-78 | 77-80 | |
| 54 | | 3-F-$C_6H_4$ | | | |
| 55 | | 4-F-$C_6H_4$ | | | |
| 56 | | 2-Cl-$C_6H_4$ | 92 | 100-103 | 87 |
| 57 | | 3-Cl-$C_6H_4$ | | | |
| 58 | | 4-Cl-$C_6H_4$ | | | |
| 59 | | 2,4-$Cl_2$-$C_6H_3$ | | | |
| 60 | 4-Br-$C_6H_4$ | 4-Br-$C_6H_4$ | | | |
| 61 | 3-Br, 4-F-$C_6H_3$ | 2-F-$C_6H_4$ | 81-84 | 65-67 | |
| 62 | " | 2-Cl-$C_6H_4$ | 125-128 | | 100-104 |
| 63 | 4-Br-$C_6H_4$ | 2-Cl-$C_6H_4$ | | | |
| 64 | " | 4-Br-$C_6H_4$ | | | |
| 65 | 4-Cl-$C_6H_4$ | 2-F-$C_6H_4$ | 80-83 | 90-92 | |
| 66 | " | 2-F, 6-Cl-$C_6H_3$ | | | |
| 67 | " | 2-Cl-$C_6H_4$ | | | |

## Patentansprüche

**1.** Verfahren zur Herstellung a,$\beta$-substituierter Acroleine der allgemeinen Formel I

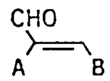

I

worin A und B gleich oder verschieden sind und für $C_1$-$C_4$-Alkyl, Naphthyl, Biphenyl oder Phenyl, das durch einen oder mehrere Halogen-, Nitro-, $C_1$-$C_4$-Alkoxy-, $C_1$-$C_4$-Halogenalkyl-, Phenoxi- oder Phenylsulfonylreste substituiert sein kann, stehen, dadurch gekennzeichnet, daß man eine Verbindung der allgemeinen Formel III

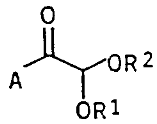

III

in der $R^1$ und $R^2$ gleich oder verschieden sind und für $C_1$- bis $C_4$-Alkyl stehen oder zusammengenommen die zur Vervollständigung eines Rings erforderlichen Kohlenstoffatome aufweisen, mit einer Phosphorverbindung der allgemeinen Formel IV oder V

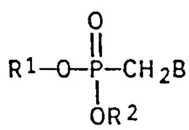

(IV)

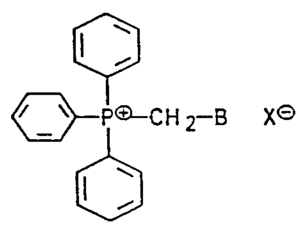

(V)

worin $X^{\ominus}$ für ein Halogenidion steht, in Gegenwart einer Base in einem Lösungsmittel bei Temperaturem von -10 bis 130 °C umsetzt.

**2.** Verfahren nach Anspruch 1, dadurch gekennzeichnet, daß man in einem polaren, aprotischen Lösungsmittel bei Temperaturen zwischen 10 und 30 °C arbeitet.

**3.** $\alpha,\beta$-substituierte Acroleine der allgemeinen Formel I, in der - wenn A Phenyl ist - B für 2-Fluorphenyl, 3-Fluorphenyl, 4-Fluorphenyl, 2-Fluor-6-Chlorphenyl, 2-Chlorphenyl oder 4-Bromphenyl steht,
wenn A 2,4-Dichlorphenyl ist, B für 2-Fluorphenyl, 3-Fluorphenyl, 4-Fluorphenyl, 2-Fluor-6-Chlorphenyl, 2-Chlorphenyl, 3-Chlorphenyl, 4-Chlorphenyl, 2,4-Dichlorphenyl oder 2,6-Dichlorphenyl steht,
wenn A 2-Chlorphenyl ist, B für 2-Fluorphenyl, 3-Fluorphenyl, 4-Fluorphenyl, 2-Chlorphenyl, 3-Chlorphenyl, 4-Chlorphenyl, 2,4-Dichlorphenyl, 2,6-Dichlorphenyl oder 4-Bromphenyl steht,
wenn A 2-Fluorphenyl ist, B für 2-Fluorphenyl, 3-Fluorphenyl, 4-Fluorphenyl, 2-Chlorphenyl, 3-Chlorphenyl, 4-Chlorphenyl oder 2,4-Dichlorphenyl steht,
wenn A 4-Fluorphenyl ist, B für 2-Fluorphenyl, 3-Fluorphenyl, 4-Fluorphenyl, 2-Fluor-4-Chlorphenyl, 2-Chlorphenyl, 3-Chlorphenyl, 2,4-Dichlorphenyl, 2,6-Dichlorphenyl, 4-Bromphenyl steht, und
wenn A 4-Chlorphenyl ist, B für 2-Fluorphenyl, 2-Fluor-6-Chlorphenyl, 2-Chlorphenyl oder 2,4-Dichlorphenyl steht.

## Claims

**1.** A process for the preparation of an $\alpha,\beta$-substituted acrolein of the general formula I

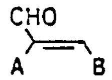

I

where A and B are identical or different and are each $C_1$-$C_4$-alkyl, naphthyl, biphenyl or phenyl which may be monosubstituted or polysubstituted by halogen, nitro, $C_1$-$C_4$-alkoxy, $C_1$-$C_4$-haloalkyl, phenoxy or phenylsulfonyl, wherein a compound of the general formula III

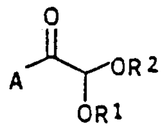

III

where $R^1$ and $R^2$ are identical or different and are each $C_1$-$C_4$-alkyl or together possess the carbon atoms required to complete a ring, is reacted with a phosphorus compound of the general formula IV or V

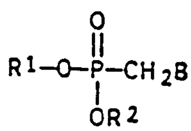

IV

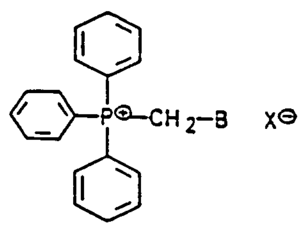

V

where $X^{\ominus}$ is a halide ion, in the presence of a base in a solvent at from -10 to 130 ° C.

**2.** A process as claimed in claim 1, wherein the reaction is carried out in a polar, aprotic solvent at from 10 to 30 ° C.

**3.** An $\alpha,\beta$-substituted acrolein of the general formula I, wherein if A is phenyl B is 2-fluorophenyl, 3-fluorophenyl, 4-fluorophenyl, 2-fluoro-6-chlorophenyl, 2-chlorophenyl or 4-bromophenyl, if A is 2,4-dichlorophenyl, B is 2-fluorophenyl, 3-fluorophenyl, 4-fluorophenyl, 2-fluoro-6-chlorophenyl, 2-chlorophenyl, 3-chlorophenyl, 4-chlorophenyl, 2,4-dichlorophenyl or 2,6-dichlorophenyl, if A is 2-chlorophenyl, B is 2-fluorophenyl, 3-fluorophenyl, 4-fluorophenyl, 2-chlorophenyl, 3-chlorophenyl, 4-chlorophenyl, 2,4-dichlorophenyl, 2,6-dichlorophenyl or 4-bromophenyl, if A is 2-fluorophenyl, B is 2-fluorophenyl, 3-fluorophenyl, 4-fluorophenyl, 2-chlorophenyl, 3-chlorophenyl, 4-chlorophenyl or 2,4-dichlorophenyl, if A is 4-fluorophenyl, B is 2-fluorophenyl, 3-fluorophenyl, 4-fluorophenyl, 2-fluoro-4-chlorophenyl, 2-chlorophenyl, 3-chlorophenyl, 2,4-dichlorophenyl, 2,6-dichlorophenyl or 4-bromophenyl, and if A is 4-chlorophenyl, B is 2-fluorophenyl, 2-fluoro-6-chlorophenyl, 2-chlorophenyl or 2,4-dichlorophenyl.

**Revendications**

**1.** Procédé de préparation d'acroléines $\alpha,\beta$-substituées de formule générale I

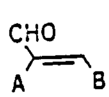

I

dans laquelle A et B sont identiques ou différents et sont mis chacun pour un groupement alkyle en $C_1$-$C_4$, naphtyle, biphényle ou phényle, qui peut être substitué par un ou plusieurs atomes d'halogène ou

restes nitro, alcoxy en $C_1$-$C_4$, halogénoalkyle en $C_1$-$C_4$, phénoxy ou phénylsulfonyle, caractérisé en ce qu'on fait réagir, en présence d'une base, dans un solvant et à une température de -10 à 130 ° C, un composé de formule générale III

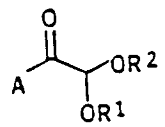

III

dans laquelle $R^1$ et $R^2$ sont identiques ou différents et sont mis pour des restes alkyle en $C_1$-$C_4$ ou, pris ensemble, comportent les atomes de carbone nécessaires pour compléter un noyau, avec un composé du phosphore de formule générale IV ou V

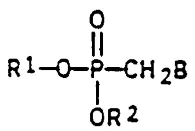

(IV)

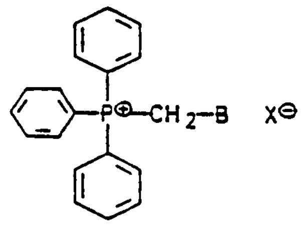

(V)

dans laquelle $X^{\ominus}$ est mis pour un ion halogénure.

2. Procédé selon la revendication 1, caractérisé en ce qu'on procède dans un solvant aprotique polaire, à des températures comprises entre 10 et 30 ° C.

3. Acroléines $\alpha,\beta$-substituées de formule générale I, dans laquelle, lorsque A est un groupement phényle, B est mis pour un groupement 2-fluorophényle, 3-fluorophényle, 4-fluorophényle, 2-fluoro-6-chlorophényle, 2-chlorophényle ou 4-bromophényle,
lorsque A est un groupement 2,4-dichlorophényle, B est mis pour un groupement 2-fluorophényle, 3-fluorophényle, 4-fluorophényle, 2-fluoro-6-chlorophényle, 2-chlorophényle, 3-chlorophényle, 4-chlorophényle, 2,4-dichlorophényle ou 2,6-dichlorophényle,
lorsque A est un groupement 2-chlorophényle, B est mis pour un groupement 2-fluorophényle, 3-fluorophényle, 4-fluorophényle, 2-chlorophényle, 3-chlorophényle, 4-chlorophényle, 2,4-dichlorophényle, 2,6-dichlorophényle ou 4-bromophényle,
lorsque A est un groupement 2-fluorophényle, B est mis pour un groupement 2-fluorophényle, 3-fluorophényle, 4-fluorophényle, 2-chlorophényle, 3-chlorophényle, 4-chlorophényle ou 2,4-dichlorophényle,
lorsque A est un groupement 4-fluorophényle, B est mis pour un groupement 2-fluorophényle, 3-fluorophényle, 4-fluorophényle, 2-fluoro-4-chlorophényle, 2-chlorophényle, 3-chlorophényle, 2,4-dichlorophényle, 2,6-dichlorophényle ou 4-bromophényle, et
lorsque A est un groupement 4-chlorophényle, B est mis pour un groupement 2-fluorophényle, 2-fluoro-6-chlorophényle, 2-chlorophényle ou 2,4-dichlorophényle.